# FASCICULE DE BREVET EUROPEEN

(11) **EP 1 006 128 B1**
(45) Date de publication et mention de la délivrance du brevet: **25.08.2004**
(21) Numéro de dépôt: 99403024.5
(22) Date de dépôt: 03.12.1999
(51) Int. Cl.: C08B 30/18

(54) **Maltodextrines branchées et leur procédé de préparation**
Verzweigte Maltodextrine und Verfahren zu deren Herstellung
Branched maltodextrins and process for their preparation

(30) Priorité: 04.12.1998 FR 9815344
(43) Date de publication de la demande: 07.06.2000
(73) Titulaire: Roquette Frères, 62136 Lestrem (FR)
(72) Inventeur: Fouache, Catherine, 62113 Sailly Labourse (FR); Duflot, Pierrick, 62136 Lacouture (FR); Looten, Philippe, 59130 Lambersart (FR)
(74) Mandataire: Boulinguiez, Didier

(56) Documents cités:
- EP-A- 0 953 578
- US-A- 4 840 807
- DATABASE CAS [Online] stn vol. 80, n° 12 , abstract n° 61369, 1974 "Dextrin production" XP002110709 & JP 48 067447 A (SENBA TOKA KOGYO K.K.) 14 septembre 1973 (1973-09-14)
- DATABASE WPI Week 9548 Derwent Publications Ltd., London, GB; AN 95-371178 XP002110711 "Prodn. of hard-to-digest starch with e.g. hydrochloric acid." & JP 07 252301 A (NAKANO SUMISE KK), 3 octobre 1995 (1995-10-03)
- DATABASE CAS [Online] stn vol. 85, n° 19, abstarct n° 141580, 1976 "Production of branched dextrin" XP002110710 & JP 51 088645 A (AJINOMOTO CO. INC.) 3 août 1976 (1976-08-03)
- PATENT ABSTRACTS OF JAPAN vol. 015, no. 345 (C-0864), 3 septembre 1991 (1991-09-03) & JP 03 137102 A (NIPPON KOONSUTAAC KK), 11 juin 1991 (1991-06-11) & CHEMICAL ABSTRACTS, vol. 115, no. 12, 23 septembre 1991 (1991-09-23) Columbus, Ohio, US; abstract no. 116723, "Preparation of roasted dextrin with low discoloration"

## Description

L'invention a pour objet des maltodextrines branchées, hydrogénées ou non, présentant des caractéristiques particulières en termes de taux de liaisons glucosidiques 1 → 6, de teneur en sucres réducteurs et de masse moléculaire moyenne.

L'invention concerne également un procédé de fabrication desdites maltodextrines branchées. Elle vise encore une composition acariogène comprenant de telles maltodextrines branchées et au moins un polyol, utilisable dans les produits destinés à être ingérés par les hommes ou les animaux.

Au sens de l'invention, on entend par maltodextrines branchées des maltodextrines dont la teneur en liaisons glucosidiques 1 → 6 est supérieure à celle des maltodextrines standards.

Les maltodextrines standards se définissent comme des mélanges purifiés et concentrés de glucose et de polymères de glucose essentiellement lié en 1 → 4 avec seulement de 4 à 5 % de liaisons glucosidiques 1 → 6, de poids moléculaires extrêmement variés, complètement solubles dans l'eau et à faible pouvoir réducteur.

Ces maltodextrines standards sont classiquement produites par hydrolyse acide ou enzymatique d'amidon de céréales ou de tubercules. La classification des maltodextrines standards repose principalement sur la mesure de leur pouvoir réducteur, exprimé classiquement par la notion de Dextrose Equivalent ou D.E.. Sur ce point particulier, la définition des maltodextrines reprise dans les *Monograph Spécifications* du *Food Chemical Codex* précise que la valeur de D.E. ne doit pas excéder 20.

Une telle mesure du D.E. est cependant insuffisante pour représenter précisément la distribution moléculaire des maltodextrines standards. En effet, l'hydrolyse acide de l'amidon, totalement aléatoire, ou son hydrolyse enzymatique, un peu plus ordonnée, fournissent des mélanges de glucose et de polymères de glucose que la seule mesure du D.E. ne permet pas de définir avec précision, et qui comportent des molécules de courte taille, de faible Degré de Polymérisation (D.P.), aussi bien que des molécules de taille très longue, de D.P. élevé.

La mesure du D.E. ne donne en fait qu'une idée approximative du D.P. moyen du mélange du glucose et des polymères de glucose constitutifs des maltodextrines standards et donc de leur masse moléculaire moyenne en nombre (Mn). Pour compléter la caractérisation de la distribution des masses moléculaires des maltodextrines standards, la détermination d'un autre paramètre est importante, celui de la masse moléculaire moyenne en poids (Mp).

Dans la pratique, les valeurs de Mn et de Mp ne se calculent pas, mais se mesurent par différentes techniques. On utilise par exemple une méthode de mesure adaptée aux polymères de glucose, qui repose sur la chromatographie de perméation de gel sur des colonnes de chromatographie étalonnées avec des pullulans de masses moléculaires connues.

Le rapport Mp/Mn est appelé indice de polymolécularité (I.P.) et permet de caractériser globalement la distribution des masses moléculaires d'un mélange polymérique. En règle générale, la répartition en masses moléculaires des maltodextrines standards conduit à des I.P. compris entre 5 et 10.

Sur le plan applicatif, les maltodextrines standards sont utilisées dans de nombreux domaines industriels et en particulier dans le domaine alimentaire.

Cependant, leur faible taux de branchement, leur teneur relativement élevée en composés de faible D.P. et le fait qu'aucun effet prébiotique ne leur soit attribué, font que les maltodextrines standards ne peuvent être utilisées dans des applications pour lesquelles il y a nécessité de disposer de composés polyglucosylés de faible pouvoir calorique, de faible cariogénicité ou améliorant la qualité de la flore intestinale.

Par "composés polyglucosylés de faible pouvoir calorique" on entend des composés polyglucosylés qui, en n'étant que faiblement assimilés par l'organisme humain ou animal ou très peu sensibles aux activités enzymatiques de l'intestin grêle, n'apportent pas le pouvoir calorique des composés polyglucosylés standards.

Par "composés polyglucosylés de faible cariogénicité" , on entend des composés qui présentent une moindre acidification par les bactéries de la cavité buccale que les sucres classiques, tels que le saccharose, le glucose, le fructose ou les composés polyglucosylés standards. L'effet cariogène est en effet dû à la présence, dans la cavité buccale, de bactéries qui métabolisent les sucres et entraînent la production d'acides. L'abaissement du pH de la bouche conduit à la dissolution de l'hydroxyapatite de l'émail dentaire et la création de cavités.

Par "améliorer la qualité de la flore intestinale" , on entend promouvoir le développement dans le gros intestin de micro-organismes bénéfiques pour la santé de l'homme ou des animaux, tels que les flores bifidogènes, butyrogènes, lactiques... On parlera également en ce cas d'effet prébiotique, car favorisant le développement d'un tel ensemble de populations de micro-organismes bénéfiques pour la santé.

De tout ce qui précède, il ressort qu'il existe un besoin non satisfait de disposer de maltodextrines, qui en plus de leurs propriétés habituelles, sont à bas pouvoir calorique, de faible cariogénicité et possèdent des capacités d'amélioration de la qualité de la microflore intestinale.

La société Demanderesse a eu le mérite de concilier tous ces objectifs réputés jusqu'alors inconciliables en imaginant et élaborant, au prix de nombreuses recherches, de nouveaux types de produits, à savoir des maltodextrines branchées particulières.

Les maltodextrines branchées conformes à l'invention sont ainsi caractérisées par le fait qu'elles présentent entre 22 et 35 %, de préférence entre 27 et 34 %, de liaisons glucosidiques 1 → 6, une teneur en sucres réducteurs inférieure à 20 %, un indice de polymolécularité inférieur à 5 et une masse moléculaire moyenne en nombre Mn au plus égale à 4500 g/mole.

En pratique, le Mn se situe généralement entre 250 et 4500 g/mole.

La teneur en liaisons glucosidiques 1 → 6 comprise entre 22 et 35 % confère aux maltodextrines branchées conformes à l'invention un caractère d'indigestibilité qui a pour consequence de diminuer leur pouvoir calorique, en empêchant leur assimilation au niveau de l'intestin grêle. Leur faible contenu en molécules de faibles D.P., tels les D.P. 1, contribue également à ce que les maltodextrines branchées conformes à l'invention présentent une plus faible caloricité que les maltodextrines standards, la quantité de glucose libre directement assimilable par l'organisme étant ainsi fortement réduite. La détermination du pouvoir calorique des maltodextrines branchées est effectuée par calcul, à partir de l'évaluation de la part représentée par la fraction indigestible dans l'intestin grêle et fermentée dans le gros intestin, considérée ici comme apportant 2kcal/g. Les maltodextrines branchées conformes à l'invention ont ainsi une valeur calorique déduite inférieure à 2,5 kcal/g.

La teneur en liaisons gluccsidiques 1 → 6 élevée a pour conséquence d'abaisser le pouvoir cariogène des maltodextrines branchées conformes à l'invention, en réduisant leur assimilation par les micro-organismes de la cavité buccale.

Ce taux élevé en liaisons glucosidiques 1 → 6, confère également à ces maltodextrines branchées des propriétés prébiotiques tout à fait particulières. En effet, il est apparu que les bactéries du caecum et du colon de l'homme et des animaux telles les bactéries butyrogènes, lactiques ou propioniques métabolisent ces composés hautement branchés.

D'autre part, les maltodextrines branchées conformes à l'invention favorisent le développement des bactéries du type bifidogène au détriment des bactéries indésirables. La détermination des effets prébiotiques des maltodextrines branchées est effectuée chez l'animal par le protocole suivant, mis au point par la société Demanderesse.

Un groupe d'animaux, préférentiellement des animaux de laboratoire (hamsters dorés de souche RJ Aura) est alimenté par une solution renfermant 15 % en poids/volume de produits à tester. Un autre groupe témoin reçoit une alimentation standard. L'essai est mené pendant 14 jours, au terme desquels les animaux sont sacrifiés et le caecum prélevé. La teneur en acides acétique, propionique, butyrique et lactique, qui illustre le développement de la microflore intestinale correspondante, est déterminée sur le surnageant d'une préparation du contenu caecal après centrifugation. Les analyses réalisées après l'assimilation de maltodextrines branchées hydrogénées de haut poids moléculaires conformes à l'invention ont par exemple montré un remarquable développement de la microflore intestinale.

Les maltodextrines branchées conformes à l'invention présentent en outre, une teneur en liaisons glucosidiques 1 → 4 résiduelles relativement élevée et un faible pouvoir réducteur qui leur permettent de conserver les mêmes fonctionnalités de base que les maltodextrines standards. Cette teneur en liaisons glucosidiques 1 → 4 peut être comprise entre 42 et 50 %, teneur qui, à la connaissance de la Société Demanderesse, n'a encore jamais été décrite en combinaison avec une teneur en liaisons glucosidiques 1 → 6 comprise entre 22 et 35 % conforme à l'invention. De manière préférentielle, les maltodextrines branchées conformes à l'invention présentent un ratio de liaisons glucosidiques 1 → 4 /1 → 6 compris entre 1,2 et 2,3 et notamment entre 1,3 et 2. Des maltodextrines branchées présentant un ratio compris entre 1,3 et 1,8 sont exemplifiées ci-après. Elles peuvent ainsi jouer le rôle d'agents texturants, épaississants et/ou gélifiants, de charge ou d'encapsulation, notamment dans les produits alimentaires, pharmaceutiques ou vétérinaires.

Les maltodextrines branchées conformes à l'invention présentent enfin un I.P. dont le contrôle à une valeur inférieure à 5 permet de définir une population de polymères de glucose de faible dispersité des masses moléculaires, tout en offrant une gamme de masses moléculaires satisfaisantes, puisque les valeurs de Mn peuvent atteindre 4500 g/mole. Cette valeur de l'IP peut notamment se situer entre 1,5 et 3, et par exemple entre 1,8 et 2,9.

Une première famille de produits conformes à l'invention est constituée par des maltodextrines branchées de hauts poids moléculaires, qui présentent de préférence une teneur en sucres réducteurs au plus égale à 5 et un Mn compris entre 2000 et 4500 g/mole.

Dans cette famille, on peut distinguer une première sous-famille constituée par des maltodextrines branchées de hauts poids moléculaires qui présentent une teneur en sucres réducteurs inférieure à 2 % et un Mn compris entre 3000 et 4500 g/mole.

Une seconde sous-famille est constituée par des maltodextrines branchées de hauts poids moléculaire qui présentent une teneur en sucres réducteurs comprise entre 2 % et 5 % et un Mn compris entre 2000 et 3000 g/mole.

Une seconde famille de produits conformes à l'invention est constituée de maltodextrines branchées de bas poids moléculaires, qui présentent de préférence une teneur en sucres réducteurs comprise entre 5 et 20 % et un Mn inférieur à 2000 g/mole.

Dans cette seconde famille, on peut distinguer une première sous-famille constituée par des maltodextrines branchées de bas poids moléculaires qui présentent une teneur en sucres réducteurs comprise entre plus de 5 % et 8 % et un Mn compris entre 500 et 1500 g/mole.

Une seconde sous-famille est constituée de maltodextrines branchées de bas poids moléculaires qui présentent une teneur en sucres réducteurs comprise entre plus de 8 % et 15 % et un Mn inférieur à 500 g/mole.

L'invention concerne aussi des maltodextrines branchées, telles que ci-avant présentées, sous forme hydrogénée. Les maltodextrines branchées hydrogénées sont notamment destinées à des applications qui nécessitent des produits de bonne stabilité thermique et où la présence de sucres réducteurs est donc à éviter.

L'invention concerne également une composition acariogène, caractérisée en ce qu'elle comprend des maltodextrines conformes à l'invention et au moins un polyol. Ledit polyol est choisi préférentiellement dans le groupe constitué par le threitol, l'érythritol, le xylitol, l'arabitol, le ribitol, le sorbitol, le mannitol, le maltitol, le maltotriitol, le maltotétraitol, le lactitol, l'isomaltulose hydrogéné, la glycérine et les hydrolysats d'amidon hydrogénés.

Selon un mode de réalisation avantageux conforme à l'invention, la composition acariogène comprend de 30 à 70 % en poids de maltodextrines branchées et de 70 à 30 % en poids de maltitol, composition qui trouve une application toute particulière dans la fabrication de gommes et autres confiseries.

De par leurs propriétés physico-chimiques et physiologiques, les maltodextrines branchées conformes à l'invention ont un intérêt certain et immédiat notamment dans la préparation de compositions acariogènes destinées à être ingérées par les hommes ou les animaux.

Par l'expression "compositions destinées à être ingérées par les hommes et les animaux" , on entend les compositions ou produits destinés à l'ingestion et à l'administration orale tels que diverses denrées alimentaires comme les confiseries, les pâtisseries, les crèmes glacées, les pâtes à mâcher, les chewing-gums, les boissons, les confitures, les soupes, les préparations à base de lait, les yaourts, les gâteaux, les aliments préparés pour animaux, les produits pharmaceutiques, vétérinaires, diététiques ou hygiéniques tels que par exemple les élixirs, les sirops contre la toux, les tablettes ou les comprimés, les pastilles, les solutions d'hygiène buccale, les pâtes et les gels dentifrices.

Pour préparer les maltodextrines branchées conformes à l'invention, on réalise la succession des étapes suivantes qui consiste en ce que l'on :
a. prépare un amidon acidifié déshydraté présentant une humidité inférieure à 5 %, de préférence inférieure ou égale à 4 %,
b. traite l'amidon acidifié ainsi déshydraté dans un réacteur de type à couche mince à une température comprise entre 120 et 300°C, de préférence comprise entre 150 et 200°C.
c. recueille, purifie et de préférence concentre les dérivés d'amidon branchés ainsi obtenus,
d. effectue un fractionnement moléculaire desdits dérivés d'amidon branchés en fonction de leur masse moléculaire moyenne en nombre de manière à obtenir les maltodextrines branchées.

La première étape du procédé conforme à l'invention consiste à préparer un amidon acidifié déshydraté présentant une humidité inférieure à 5 %, de préférence inférieure ou égale à 4 %.

L'origine botanique de l'amidon est indifférente. L'amidon peut donc provenir du blé, du maïs, de la pomme de terre, du riz ou du manioc. Cependant on préfère avantageusement mettre en oeuvre l'amidon de blé, comme exemplifié ci-après.

L'acide utilisé pour acidifier l'amidon peut-être choisi dans le groupe constitué de l'acide chlorhydrique, l'acide sulfurique, l'acide phosphorique, l'acide nitrique et l'acide citrique. Cependant, compte tenu du fait que l'acide citrique est susceptible de générer des liaisons esters indésirables, car responsables d'amertume et que la manutention de l'acide sulfurique pose d'évidents problèmes de sécurité, on préfère utiliser dans le cadre de l'invention l'acide chlorhydrique, l'acide phosphorique ou l'acide nitrique. La quantité d'acide mise en oeuvre dans le procédé conforme à l'invention est comprise entre 5 et 100 meq H+ /kg d'amidon sec, et de préférence comprise entre 10 et 50 meq H+/kg d'amidon sec. Il est important que la répartition de l'acide dans l'amidon soit la plus homogène possible.

Différentes techniques peuvent être mises en oeuvre pour l'acidification de l'amidon, comme l'acidification continue ou discontinue, en phase sèche ou liquide. Néanmoins, si l'amidon acidifié est destiné à être mis en oeuvre dans un procédé de modification en continu, on préfère dans la présente invention utiliser un moyen d'acidification en continu pour réaliser un procédé le plus continu possible, et limiter ainsi les opérations non productives (chargement, déchargement, vidange).

L'acidification réalisée, l'amidon est séché jusqu'à une humidité inférieure à 5 % afin de favoriser ultérieurement les branchements ou ramifications entre molécules. Lors de cette étape de séchage, il convient également de limiter les réactions d'hydrolyse, afin d'éviter l'augmentation de sucres réducteurs indésirables.

La société Demanderesse a pu mettre en évidence qu'il fallait privilégier, lors de cette étape, des techniques de séchage de type continu permettant d'atteindre l'humidité recherchée en un temps de séjour de l'ordre de la minute, voire de la seconde et ainsi limiter les réactions d'hydrolyse de l'amidon.

La deuxième étape du procédé conforme à l'invention consiste à traiter l'amidon acidifié et déshydraté dans un réacteur de type continu à couche mince à une température comprise entre 120 et 300°C, de préférence comprise entre 150 et 200°C. Par réacteur de type continu à couche mince, la Société Demanderesse entend tout type de réacteur permettant d'appliquer au produit une température très élevée pendant un temps très court, afin d'obtenir une transformation importante de la structure du produit, principalement au niveau des liaisons glucosidiques, en générant simultanément le moins de produits de dégradation possible. Les réacteurs de type turbosécheur (par exemple commercialisés par la Société VOMM) ou malaxeur (par exemple commercialisés par la Société BÜSS) répondent à cette définition.

Avantageusement, ce réacteur de type continu à couche mince est constitué d'une zone réactionnelle maintenue à une température comprise entre 150 et 200°C. Le chauffage peut être assuré par convection, conduction ou rayonnement. Eventuellement, cette zone réactionnelle peut être précédée et/ou suivie d'une zone de convoyage.

Le temps de séjour de l'amidon acidifié déshydraté dans ce réacteur de type continu à couche mince est fonction de la température et de la quantité d'acide mise en oeuvre lors de l'étape d'acidification. Le traitement à haute température dans ces conditions et dans une très courte période de temps qui ne dépasse généralement pas la dizaine de secondes conduit à l'obtention de composés dérivés de l'amidon très partiellement hydrolysés et surtout enrichis en liaisons glucosidiques 1 → 6 résultant des réactions de ramification.

La troisième étape du procédé conforme à l'invention consiste à recueillir, purifier et de préférence concentrer les dérivés d'amidon branchés ainsi obtenus. On rassemble les dérivés d'amidon branchés en sortie du réacteur et on met en oeuvre des étapes de purification classiques pour éliminer les impuretés de nature protéique, lipidique, voire des matières colorantes. Cette purification est menée par toute méthode connue en soi par l'homme du métier, par exemple par filtration, traitement au noir, décoloration et déminéralisation sur résines, voire par ultrafiltration. La filtration peut-être réalisée en deux temps, sous vide, à l'aide d'un filtre rotatif sous vide, et sous pression, à l'aide d'un filtre sous pression.

La décoloration peut suivre l'enchaînement classique sur résines adsorbantes non fonctionnalisées de ROHM et HAAS (de type XAD 16), et sur résines cationiques de Purolite (de type C 145) et anioniques de Purolite (de type A 860). Ensuite, les dérivés d'amidon branchés filtrés et décolorés peuvent être concentrés par toute technique connue en soi, par exemple par évaporation.

On soumet ensuite les dérivés d'amidon branchés a un fractionnement moléculaire, puis éventuellement à une hydrogénation catalytique. On peut également inverser l'ordre de ces deux étapes. Cette étape de fractionnement moléculaire peut consister, par exemple, en une séparation chromatographique, en une séparation sur membrane ou en une précipitation sélective par solvant.

Cette étape de fractionnement moléculaire est destinée à recueillir les fractions de maltodextrines branchées qui présentent des caractéristiques pouvant être adaptées à une application donnée, en termes de pouvoir calorique réduit, de faible cariogénicité, ou de propriétés prébiotiques.

Ainsi, des investigations de la société Demanderesse ont permis de montrer que, pour la fabrication de gommes, la fraction de maltodextrines branchées présentant un Mn se situant entre 2000 et 4000 g/mole donnait de très bons résultats.

De façon générale, le fractionnement moléculaire est effectué sur les dérivés d'amidon branchés filtrés puis déminéralisés et concentrés jusqu'à une matière sèche pratiquement comprise entre 20 et 70 %, de préférence entre 25 et 60 %.

Lorsque l'on procède à ce fractionnement moléculaire par voie chromatographique, l'étape de fractionnement chromatographique est effectuée de manière connue en soi, de manière discontinue ou continue (lit mobile simulé), sur des résines cationiques fortes de type macroporeuse, chargées préférentiellement à l'aide d'ions alcalins ou alcalino-terreux tels que le calcium et le magnésium mais plus préférentiellement à l'aide d'ions sodium ou potassium.

Selon un mode de réalisation préféré, le fractionnement chromatographique est réalisé en employant les procédé et appareillage décrits dans le brevet américain n° 4 422 881 dont la société Demanderesse est titulaire. L'obtention des maltodextrines branchées est ainsi avantageusement réalisée par passage des dérivés d'amidon branchés sur une résine polystyrène / divinyl benzène (ou DVB), de type cationique forte macroporeuse, sous forme potassium, de granulométrie 250 à 350 µm. La résine polystyrénique cationique forte macroporeuse, sous forme potassium est choisie de préférence dans le groupe constitué de la C 141 de Purolite à 5 % de DVB, de la C 145 de Purolite à 8 % de DVB ou de la C 150 de Purolite à 12 % de DVB.

De manière générale, la chromatographie est réalisée sur 4 à 10 plateaux maintenus à une température de l'ordre de 80°C et l'alimentation est assurée avec un sirop de dérivés d'amidon branchés amené à une valeur d'environ 50 % de matière sèche.

Le choix des paramètres de conduite du fractionnement chromatographique est à la portée de l'homme du métier. Le choix de ces paramètres est effectué de telle manière que la fraction contenant les maltodextrines branchées présente un Mn et une teneur en liaisons 1 → 6 conformes à l'invention.

Dans le cas où cela s'avérerait nécessaire, les maltodextrines branchées conformes à l'invention peuvent, à ce stade du procédé, être soumises à une étape supplémentaire d'élimination du glucose. Ce traitement supplémentaire d'élimination du glucose peut être entrepris si le contenu limité en glucose résiduel libre des maltodextrines branchées se révèle encore indésirable pour les applications visées. On utilisera ce traitement supplémentaire par exemple dans le cas où une faible valeur calorique est recherchée pour les maltodextrines branchées, ce qui est le cas pour les maltodextrines de bas poids moléculaires.

On réalise ce traitement supplémentaire par tout moyen connu en soi, par exemple par transformation du glucose suivie d'une étape de désacidification sur échangeur d'anions. Cette étape supplémentaire peut être effectuée par transformation biologique du glucose par oxydation enzymatique ou à l'aide d'une bactérie oxydante, ou par séparation du glucose sur résine ou membrane. Elle peut être aussi effectuée à l'aide de levures qui transforment le glucose en alcool qui s'élimine par évaporation dans la suite du procédé.

Dans une variante du procédé selon l'invention, les maltodextrines branchées sont soumises à une étape d'hydrogénation catalytique. L'hydrogénation catalytique de ces maltodextrines branchées s'effectue conformément aux règles de l'art.

Les paramètres analytiques de chacune des maltodextrines branchées sont ensuite déterminés. Plus particulièrement, on détermine la teneur en sucres réducteurs, exprimée en glucose, en poids par rapport au poids sec de produit analysé, par la méthode de BERTRAND. Les valeurs de Mn et de Mw sont ensuite mesurées par chromatographie d'exclusion stérique, fondée sur la rétention sélective des molécules du soluté en fonction de leur taille, en raison de leur pénétration ou non dans les pores de la phase stationnaire. La détermination de la teneur en liaisons glucosidiques 1 → 2, 1 → 3, 1 → 4 et 1 → 6 est effectuée par l'utilisation de la technique classique de méthylation décrite dans HAKOMORI, S. , 1964, J. Biol. Chem, 55, 205.

D'autres caractéristiques et avantages de l'invention apparaîtront clairement à la lecture des exemples qui suivent. Ils ne sont toutefois donnés ici qu'à titre illustratif et non limitatif.

### EXEMPLE 1

De l'amidon de blé est acidifié par de l'acide chlorhydrique à raison de 19,6 meq H+/kg sec, puis séché à une humidité résiduelle de 4%. Cette matière première est alors introduite dans un malaxeur BUSS de type PR 46 maintenu à une température de 180°C, à un débit de 20 kg/h, avec un temps de séjour de 5 secondes. On stoppe rapidement la réaction par pulvérisation d'eau froide à 15°C.

Après purification par filtration, décoloration sur résines adsorbantes et sur résines cationiques et anioniques, les dérivés d'amidon branchés ainsi obtenus sont ramenés à une matière sèche de 50 %.

Le produit obtenu est soumis à une chromatographie sur résine cationique forte macroporeuse de Purolite C 145 sous forme potassium, de granulométrie 250-350 µm, configurée en 6 plateaux de 200 litres, maintenus à 75°C.

Les débits d'alimentation du sirop de dérivés d'amidon branchés et de l'eau d'élution sont fixés à 60 l/h et 400 l/h, au niveau du premier et du troisième plateau, respectivement. Le choix des débits de sortie du deuxième et du quatrième plateau conditionne l'obtention des fractions de maltodextrines branchées de haut poids moléculaire et de bas poids moléculaire.

On fixe le débit en sortie du deuxième plateau à 280 l/h pour obtenir le composé A et à 320 l/h pour obtenir le composé B. On fixe le débit en sortie du quatrième plateau à 180 l/h pour le composé C.

Les résultats d'analyse, après chromatographie, sont regroupés dans le tableau I suivant. A titre de comparaison, sont regroupés également certains paramètres analytiques des maltodextrines standards de l'état de la technique dont le Mn est équivalent à certaines maltodextrines branchées (composés D et E).

**TABLEAU I**

| **PRODUITS** | **A** | **B** | **C** | **D** | **E** |
|---|---|---|---|---|---|
| Sucres Réducteurs (%) | 1,75 | 2,3 | 7,2 | 5,7 | 18,2 |
| Mn (g/mole) | 3700 | 2480 | 990 | 3480 | 1140 |
| Mw (g/mole) | 5950 | 5160 | 2200 | 33075 | 10240 |
| I.P. | 1,6 | 2,1 | 2,2 | 9,5 | 9 |
| Liaison 1,2 (%) | 11 | 10 | 10 | 0 | 0 |
| Liaison 1,3 (%) | 13 | 12 | 10 | 0 | 0 |
| Liaison 1,4 (%) | 48 | 49 | 50 | 95 | 95 |
| Liaison 1,6 (%) | 28 | 29 | 30 | 5 | 5 |

### EXEMPLE 2

Les premières étapes de préparation de la maltodextrine branchée conforme à l'invention sont les mêmes que celles décrites dans l'exemple 1.

L'isolement de la fraction de bas poids moléculaire s'effectue au niveau du quatrième plateau avec un débit de sortie fixé à 140 l/h. On obtient la fraction de Mn valant 370 g/mole avec un réglage les paramètres chromatographiques fixant le rendement à 30%. Le rendement s'entend ici de la proportion de matière sèche extraite de cette fraction de haut poids moléculaire par rapport à la matière sèche introduite dans le système chromatographique.

La teneur élevée en sucres réducteurs (supérieure à 20%) a conditionné la mise en oeuvre d'une étape d'élimination du glucose, consistant à une étape d'élimination du glucose par la glucose oxydase dans les conditions suivantes : la solution ajustée à 20 % de matière sèche, pH 6,0, et chauffée à 35°C, est additionnée de Novozym 771 (glucose oxydase NOVO) à raison de 1 ml par litre de solution, soit 0,5 %/sec ; maintenue sous une agitation à 750 µm et une aération de 1,2 vvm, la solution contenant initialement 13 %/sec de glucose, titre après 5 heures, moins de 10 %/sec. Les résultats analytiques de la maltodextrine branchée F résultante conforme à l'invention sont regroupés dans le tableau II.

**TABLEAU II**

| **PRODUITS** | **F** |
|---|---|
| Sucres Réducteurs (%) | 9,9 |
| Mn (g/mole) | 380 |
| Mw (g/mole) | 755 |
| I.P. | 2 |
| Glucose libre (%/sec) | <1 |
| Liaison 1,2 (%) | 11 |
| Liaison 1,3 (%) | 12 |
| Liaison 1,4 (%) | 44 |
| Liaison 1,6 (%) | 33 |

### EXEMPLE 3

On fabrique des gommes à partir d'un mélange M de 50 % en poids de maltodextrines branchées conformes à l'invention correspondant au produit B de l'exemple 1 avec 50 % en poids de maltitol.

La formule des gommes est donnée dans le tableau III suivant.

**Tableau III**

| Formule | Pour 100 % total |
|---|---|
| Mélange M (M.S.*) | 42,8 % |
| Gomme arabique (M.S.) | 38 % |
| Menthe | 6,2 % |
| NH₄Cl | 3 % |
| Humidité relative | 10 % |

| | |
|---|---|
| * M.S. : matière sèche | |

Les caractéristiques des gommes obtenues sont regroupées dans le tableau IV suivant :

**Tableau IV**

| Gommes | |
|---|---|
| Activité en eau à 20 % | 0,57 |
| % d'humidité après 3 j à 75 % d'humidité relative à 20°C | 3,2 % |
| Caractère "collant" après 3 j sous une humidité relative de 75 %, à 20°C | 0/+ (a) |
| Dureté INSTRON | 31 |
| Dureté estimée par analyse sensorielle | +++ (b) |
| (a) 0 : absence de caractère "collant" + : léger caractère "collant" (b) consistance dure mais absence de caractère cassant | |

Le comportement des gommes obtenues avec le mélange M selon l'invention est donc tout-à-fait avantageux puisqu'il se caractérise par :
- une faible hygroscopicité, qui se traduit par une très faible reprise en eau (notamment avec une activité en eau inférieure à 3,5) lorsque ces gommes sont placées en atmosphère humide,
- une texture en bouche, et une dureté INSTRON (comprise entre 25 et 35), qui permet de mettre en oeuvre une quantité moindre de gomme arabique, d'où un gain financier appréciable.

Il ressort de tout ce qui précède que l'utilisation des maltodextrines branchées et des compositions conformes à l'invention est particulièrement adaptée à la préparation de confiseries et notamment de gommes.

A la connaissance de la Société Demanderesse, il n'a de plus jamais été décrit des gommes à base de maltodextrines branchées, présentant simultanément les deux caractéristiques précitées de reprise en eau et de dureté INSTRON.

## Revendications

1. Maltodextrines branchées, **caractérisées par le fait qu'**elles présentent entre 22 % et 35 % de liaisons glucosidiques 1 → 6, une teneur en sucres réducteurs inférieure à 20 %, un indice de polymolécularité inférieur à 5 et une masse moléculaire moyenne en nombre Mn au plus égale à 4500 g/mole.

2. Maltodextrines branchées selon la revendication 1, **caractérisées par le fait qu'**elles présentent une teneur en sucres réducteurs inférieure à 2 % et un Mn compris entre 3000 et 4500 g/mole.

3. Maltodextrines branchées selon la revendication 1, **caractérisées par le fait qu'**elles présentent une teneur en sucres réducteurs comprise entre 2 % et 5 % et un Mn compris entre 2000 et 3000 g/mole.

4. Maltodextrines branchées selon la revendication 1, **caractérisées par le fait qu'**elles présentent une teneur en sucres réducteurs supérieure à 5 % et au plus égale à 8 % et un Mn compris entre 500 et 1500 g/mole.

5. Maltodextrines branchées selon la revendication 1, **caractérisées par le fait qu'**elles présentent une teneur en sucres réducteurs supérieure à 8 % et au plus égale à 15 % et un Mn inférieur à 500 g/mole.

6. Maltodextrines branchées selon l'une quelconque des revendications 1 à 5, **caractérisées par le fait qu'**elles sont hydrogénées.

7. Composition acariogène, **caractérisée en ce qu'**elle comprend des maltodextrines branchées selon l'une quelconque des revendications 1 à 6 et au moins un polyol.

8. Composition acariogène selon la revendication 7 **caractérisée en ce qu'**elle comprend de 30 à 70 % en poids de maltodextrines branchées conformes à l'une quelconque des revendications 1 à 6 et de 70 à 30 % en poids de maltitol.

9. Procédé de préparation de maltodextrines branchées selon l'une quelconque des revendications 1 à 6, **caractérisé par le fait que** l'on :
a. prépare un amidon acidifié déshydraté présentant une humidité inférieure à 5 %,
b. traite l'amidon acidifié ainsi déshydraté dans un réacteur de type continu à couche mince, à une température comprise entre 120 et 300°C,
c. recueille, purifie et de préférence concentre les dérivés d'amidon branchés ainsi obtenus,
d. effectue un fractionnement moléculaire desdits dérivés d'amidon branchés en fonction de leur masse moléculaire moyenne en nombre de manière à obtenir les maltodextrines branchées.

10. Procédé selon la revendication 9, **caractérisé par le fait que** l'on effectue une hydrogénation catalytique des maltodextrines branchées conformes à l'invention.

## Patentansprüche

1. Verzweigte Maltodextrine, **dadurch gekennzeichnet, daß** sie zwischen 22 % und 35 % Glucosidbindungen 1 → 6, einen Gehalt an reduzierenden Zuckern von weniger als 20 %, einen Polymolekularitätsindex von weniger als 5 und eine molare Masse als Zahlenmittelwert Mn von höchstens gleich 4500 g/mol aufweisen.

2. Verzweigte Maltodextrine nach Anspruch 1, **dadurch gekennzeichnet, daß** sie einen Gehalt an reduzierenden Zuckern von weniger als 2 % und ein Mn zwischen 3000 und 4500 g/mol aufweisen.

3. Verzweigte Maltodextrine nach Anspruch 1, **dadurch gekennzeichnet, daß** sie einen Gehalt an reduzierenden Zuckern zwischen 2 % und 5 % und ein Mn zwischen 2000 und 3000 g/mol aufweisen.

4. Verzweigte Maltodextrine nach Anspruch 1, **dadurch gekennzeichnet, daß** sie einen Gehalt an reduzierenden Zuckern von mehr als 5 % und höchstens gleich 8 % und ein Mn zwischen 500 und 1500 g/mol aufweisen.

5. Verzweigte Maltodextrine nach Anspruch 1, **dadurch gekennzeichnet, daß** sie einen Gehalt an reduzierenden Zucker von mehr als 8 % und höchstens gleich 15 % und ein Mn von weniger als 500 g/mol aufweisen.

6. Verzweigte Maltodextrine nach einem der Ansprüche 1 bis 5, **dadurch gekennzeichnet, daß** sie hydriert sind.

7. Akariogene Zusammensetzung, **dadurch gekennzeichnet, daß** sie verzweigte Maltodextrine nach einem der Ansprüche 1 bis 6 und mindestens ein Polyol enthält.

8. Akariogene Zusamamensetzung nach Anspruch 7, **dadurch gekennzeichnet, daß** sie 30 bis 70 Gew.-% verzweigte Maltodextrine nach einem der Ansprüche 1 bis 6 und 70 bis 30 Gew.-% Maltitol enthält.

9. Verfahren zur Herstellung von verzweigten Maltodextrinen nach einem der Ansprüche 1 bis 6, **dadurch gekennzeichnet, daß** man:
a. eine dehydratisierte angesäuerte Stärke mit einer Feuchtigkeit von weniger als 5 % herstellt,
b. die auf diese Weise dehydratisierte angesäuerte Stärke in einem kontinuierlichen Dünnschichtreaktor bei einer Temperatur zwischen 120 und 300°C behandelt,
c. die auf diese Weise erhaltenen verzweigten Stärkederivate gewinnt, reinigt und vorzugsweise konzentriert,
d. eine molekulare Fraktionierung dieser verzweigten Stärkederivate in Abhängigkeit von ihrer molaren Masse als Zahlenmittelwert durchführt, so daß man die verzweigten Maltodextrine erhält.

10. Verfahren nach Anspruch 9, **dadurch gekennzeichnet, daß** man eine katalytische Hydrierung der erfindungsgemäßen verzweigten Maltodextrine vornimmt.

## Claims

1. Branched maltodextrins, **characterized in that** they have between 22% and 35% glucosidic linkages 1 → 6, a content in reducing sugars lower than 20%, a polymolecularity index lower that 5, and a number average molecular weight Mn at most equal to 4500 g/mole.

2. Branched maltodextrins according to claim 1, **characterized in that** they have a content in reducing sugars lower than 2% and an Mn of between 3000 and 4500 g/mole.

3. Branched maltodextrins according to claim 1, **characterized in that** they have a content in reducing sugars between 2% and 5%, and an Mn of between 2000 and 3000 g/mole.

4. Branched maltodextrins according to claim 1, **characterized in that** they have a content in reducing sugars greater than 5% and at most equal to 8%, and an Mn of between 500 and 1500 g/mole.

5. Branched maltodextrins according to claim 1, **characterized in that** they have a content in reducing sugars greater than 8% and at most equal to 15%, and an Mn of lower than 500 g/mole.

6. Branched maltodextrins according to any one of claims 1-5, **characterized in that** they are hydrogenated.

7. Acariogenic composition **characterized in that** it comprises branched maltodextrins according to any one of claims 1-6, and at least one polyol.

8. Acariogenic composition according to claim 7, **characterized in that** it comprises between 30 and 70% by weight branched maltodextrins according to any one of claims 1-6, and between 70 and 30% by weight maltitol.

9. Process for preparing branched maltodextrins according to any one of claims 1-6, **characterized in that**:
a) a dehydrated acidified starch is prepared presenting a humidity lower than 5%,
b) the acidified starch thus dehydrated is processed in a reactor of the thin-layer continuous type, at a temperature of between 120 and 300°C,
c) the branched starch derivatives thus obtained are collected, purified and preferably concentrated,
d) the branched starch derivatives are molecular fractionated depending on their number average molecular weight, in such a way as to obtain the branched maltodextrins.

10. Process according to claim 9, **characterized in that** a catalytic hydrogenation of the branched maltodextrins according to the invention is carried out.
